(19) Europäisches Patentamt
European Patent Office
Office européen des brevets

(11) Veröffentlichungsnummer: **0 214 437**
**B1**

(12) **EUROPÄISCHE PATENTSCHRIFT**

(45) Veröffentlichungstag der Patentschrift: **12.09.90**

(21) Anmeldenummer: **86110446.1**

(22) Anmeldetag: **29.07.86**

(51) Int. Cl.[5]: **C 07 D 215/54,**
C 07 D 215/18,
C 07 D 215/24,
C 07 D 215/36,
C 07 D 215/14,
C 07 D 221/04,
C 07 D 401/04,
C 07 D 409/04, A 61 K 31/47
// C07D311/74, C07D311/94,
C07D405/04

(54) **Verfahren zur Herstellung von 1,4-Dihydropyridinderivaten über neue Zwischenprodukte.**

(30) Priorität: **09.08.85 DE 3528602**

(43) Veröffentlichungstag der Anmeldung:
**18.03.87 Patentblatt 87/12**

(45) Bekanntmachung des Hinweises auf die Patenterteilung:
**12.09.90 Patentblatt 90/37**

(84) Benannte Vertragsstaaten:
**AT BE CH DE FR GB IT LI NL SE**

(56) Entgegenhaltungen:
**EP-A-0 071 819**
**DE-A-2 018 738**

**BULLETIN OF THE CHEMICAL SOCIETY OF JAPAN, Band 42, 1969, Seiten 220-223; A. SAKURAI et al.: "The cyclization of benzoylacetonitrile and ketones by ammonium acetate"**

(73) Patentinhaber: **BAYER AG**
**D-5090 Leverkusen 1 Bayerwerk (DE)**

(72) Erfinder: **Meyer, Horst, Dr.**
**Henselweg 11**
**D-5600 Wuppertal 1 (DE)**
Erfinder: **Franckowiak, Gerhard, Dr.**
**Henselweg 10**
**D-5600 Wuppertal 1 (DE)**
Erfinder: **Günther, Thomas, Dr.**
**c/o BAYER ITALIA S.p.A., Via della Groane 126**
**I-20024 Garbagnate / Milano (IT)**
Erfinder: **Schramm, Matthias, Dr.**
**Paffrather Strasse 38**
**D-5000 Köln 80 (DE)**
Erfinder: **Kayser, Michael, Dr.**
**Fleyerstrasse 231**
**D-5800 Hagen (DE)**
Erfinder: **Bechem, Martin, Dr.**
**Obere Bergerheide 4**
**D-5600 Wuppertal 1 (DE)**
Erfinder: **Gross, Rainer, Dr.**
**Platzhofstrasse 23**
**D-5600 Wuppertal 1 (DE)**

**EP 0 214 437 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:

CHEMICAL ABSTRACTS, Band 101, Nr. 25, 17. Dezember 1984, Seite 751, Zusammenfassung Nr. 230323r, Columbus, Ohio, US; S. MARCHALIN et al.: "Dihydropyridines. LVIII. Cyclocondensation reactions of 4-substituted-benzylidene-4-phenylbenzoylacetonitriles with cycloalkanones"

J.ORG.CHEM. Vol. 46, Seiten 3823-30, (1981)

J.CHEM.SOC (C), Seiten 2377-82, (1970)

J.CHEM.SOC (C), Seiten 771-774, (1970)

## Beschreibung

Die Erfindung betrifft ein Verfahren zur Herstellung von 1,4-Dihydropyridinderivaten unter Verwendung von neuen Zwischenprodukten sowie deren Herstellung.

Aus J. Org. Chem. Vol. 46, (1981), S. 3823—3830 und aus J. Chem. Soc. (C) (1970), S. 2377—2382 sind bereits Phenyl-substituierte 8-Morpholino-4H-chromene bekannt, die sich jedoch strukturell eindeutig von den erfindungsgemäßen Alkyl-substituierten Verbindungen unterscheiden. Weiterhin geben diese Publikationen keinen Hinweis auf die Verwendung zur Herstellung von 1,4-Dihydropyridinen.

Es ist bekannt, daß man durch Umsetzung von Benzylidencyclohexanon und Morpholinocyclohexen ein Cycloaddukt erhält, das durch Hydrolyse und anschließende Behandlung mit Ammoniak nicht das Dihydropyridin, sondern lediglich dessen Oxidationsprodukt ergibt [I. W. Lewis, P. L. Meyers, M. I. Readhead, J. Chem. Soc. (1970), 771]. Bei Kenntnis dieses Standes der Technik war davon auszugehen (s. Seite 772), daß in Nebenreaktionen Pyrane entstehen.

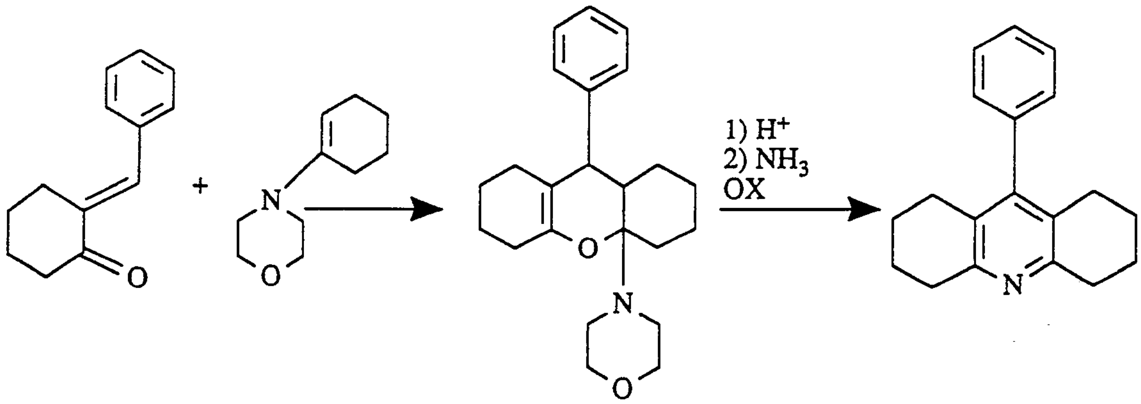


Es konnte daher nicht erwartet werden, daß man bei der Umsetzung von cyclischen tertiären Enaminen mit Benzylidenacetessigestern oder Benzylidennitroacetonen 1,4-Dihydropyridinderivate erhält, die unter den Versuchsbeindungen stabil sind und nicht zu den entsprechenden Pyridinen oxidiert werden.

Die Erfindung betrifft ein Verfahren zur Herstellung von 1,4-Dihydropyridinen der allgemeinen Formel (I)

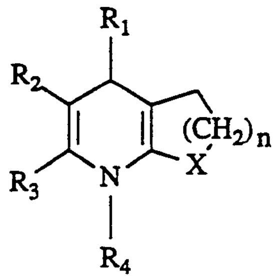

(I)

in welcher

$R_1$ für Phenyl, Thienyl, Furyl, Pyridyl, Pyrimidyl, Benzoxadiazolyl oder Thiochromenyl steht, wobei die genannten Reste gegebenenfalls 1 bis 2 gleiche oder verschiedene Substituenten aus der Gruppe $C_1$—$C_4$-Alkyl, $C_1$—$C_4$-Alkoxy, $C_1$—$C_4$-Alkylthio, Fluor, Chlor, Nitro, Cyano, Hydroxy, Trifluormethyl, Trifluormethoxy, Difluormethoxy, Fluormethoxy, Phenyl, Benzyl, Benzyloxy oder Benzylthio enthalten,

$R_2$ für Wasserstoff, Nitro, Cyano, $C_1$—$C_4$-Alkylsulfonyl oder für die Gruppe

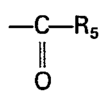 steht

wobei

$R_5$ für geradkettiges oder verzweigtes $C_1$—$C_4$-Alkyl steht, oder für eine Gruppe der Formel —O—$R_6$ steht, wobei

$R_6$ geradkettiges, verzweigtes oder cyclisches $C_1$—$C_6$-Alkyl darstellt, das gegebenenfalls durch ein Sauerstoffatom in der Kette unterbrochen ist und das gegebenenfalls substituiert ist durch ein oder mehrere Fluor, Hydroxy, Cyano, Phenyl oder Benzylmethylamino,

$R_3$ für geradkettiges oder verzweigtes $C_1$—$C_4$-Alkyl steht, das gegebenenfalls substituiert ist durch Hydroxy, Chlor, Brom oder Acetyloxy,

$R_4$ für Wasserstoff oder geradkettiges oder verzweigtes $C_1$—$C_4$-Alkyl steht,

X für die Gruppe =C=O oder für eine direkte Bindung steht und

n für eine Zahl von 1 bis 12 steht,

in Form ihrer Isomeren, Isomerengemische, Racematen und optischen Antipoden sowie ihrer physiologisch unbedenklichen Salze, dadurch gekennzeichnet, daß man Verbindungen der allgemeinen Formel (IV)

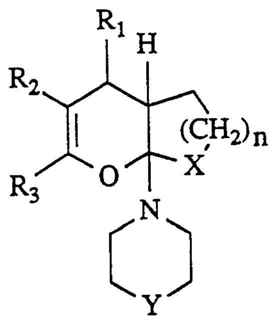

(IV)

in welcher

$R_1$, $R_2$, $R_3$, X und n die oben angegebenen Bedeutung haben, und

Y für eine direkte Bindung, für Sauerstoff, NH, Methylen oder Ethylen steht mit Aminen der allgemeinen Formel (V)

$$R_4—NH_2 \qquad (V)$$

in welcher

$R_4$ die oben angegebene Bedeutung hat

oder mit deren Additionsprodukten in Anwesenheit von Säuren und gegebenenfalls in Anwesenheit von Wasser oder inerten organischen Lösungsmitteln umsetzt.

Physiologisch unbedenkliche Salze der erfindungsgemäßen Stoffe können Salze mit anorganischen oder organischen Säuren sein. Als Beispiele seien genannte: Halogenide wie Bromide, Chloride, Hydrogensulfate, Sulfate, Hydrogenphosphate, Acetate, Maleate, Fumarate, Citrate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Herstellung von Antipoden als auch von Racemformen sowie Diastereomerengemischen. Die Racemformen lassen sich ebenso wie die Diastereomeren in bekannter Weise in die stereoisomer einheitlichen Bestandteile trennen (vgl. E. L. Eliel, Stereochemistry of Carbon Compounds, McGraw Hill, 1962).

Die Erfindung betrifft ebenfalls Verbindungen der allgemeinen Formel (IV)

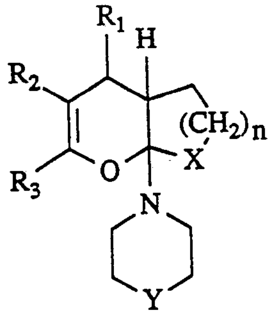

(IV)

in welcher

$R_1$ für Phenyl, Thienyl, Furyl, Pyridyl, Pyrimidyl, Benzoxadiazolyl oder Thiochromenyl steht, wobei die genannten Reste gegebenenfalls 1 bis 2 gleiche oder verschiedene Substituenten aus der Gruppe $C_1$—$C_4$-

Alkyl, $C_1$—$C_4$-Alkoxy, $C_1$—$C_4$-Alkylthio, Fluor, Chlor, Nitro, Cyano, Hydroxy, Trifluormethyl, Trifluormethoxy, Difluormethoxy, Fluormethoxy, Phenyl, Benzyl, Benzyloxy oder Benzylthio enthalten,

$R_2$ für Wasserstoff, Nitro, Cyano, $C_1$—$C_4$-Alkylsulfonyl oder für die Gruppe

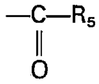 steht

wobei

$R_5$ für geradkettiges oder verzweigtes $C_1$—$C_4$-Alkyl steht, oder für eine Gruppe der Formel —O—$R_6$ steht, wobei

$R_6$ geradkettiges, verzweigtes oder cyclisches $C_1$—$C_6$-Alkyl darstellt, das gegebenenfalls durch ein Sauerstoffatom in der Kette unterbrochen ist und das gegebenenfalls substituiert ist durch ein oder mehrere Fluor, Hydroxy, Cyano, Phenyl oder Benzylmethylamino,

$R_3$ für geradkettiges oder verzweigtes $C_1$—$C_4$-Alkyl steht, das gegebenenfalls substituiert ist durch Hydroxy, Chlor, Brom oder Acetyloxy,

X für die Gruppe

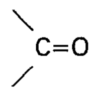

oder für eine direkte Bindung steht, und

n für eine Zahl von 1 bis 12 steht, und

Y für eine direkte Bindung, für Sauerstoff, NH, Methylen oder Ethylen steht,

sowie deren Verwendung bei der Herstellung von 1,4-Dihydropyridinen der allgemeinen Formel (I).

Die erfindungsgemäßen Verbindungen der Formel (IV) können dadurch hergestellt werden, daß man Benzylidenverbindungen der allgemeinen Formel II

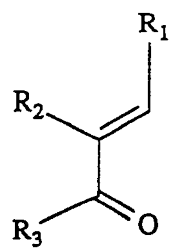

(II)

in welcher

$R_1$—$R_3$ die oben angegebene Bedeutung haben,

und Enamine der allgemeinen Formel (III)

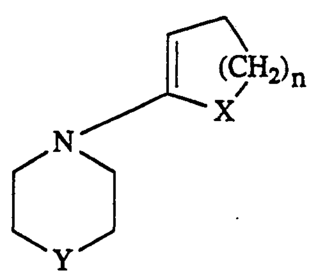

(III)

in welcher

X und n die oben angegebene Bedeutung haben und

Y für eine direkte Bindung,

für Sauerstoff, Schwefel, Amino, $C_1$—$C_4$-Alkyl-amino, oder

für eine Methylenkette mit 1 bis 2 Kohlenstoffatomen steht,

gegebenenfalls in Gegenwart von Wasser und/oder inerten organischen Lösungsmitteln zu den Zwischenprodukten der allgemeinen Formel (IV)

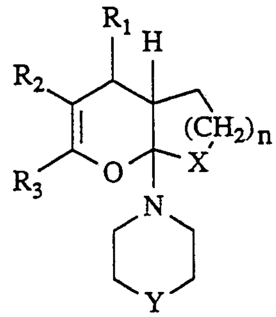

(IV)

in welcher

$R_1$—$R_3$, X, Y und n die oben angegebene Bedeutung haben, umsetzt.

Diese neuen Zwischenprodukte der allgemeinen Formel (IV) treffen sich dann ihrerseits mit Aminen der allgemeinen Formel (V)

$$R_4—NH_2 \qquad (V)$$

in welcher

$R_4$ die oben angegebene Bedeutung hat,

oder mit deren Additionsprodukten, in Gegenwart von Säuren und gegebenenfalls in Anwesenheit von Wasser und/oder inerten organischen Lösungsmitteln zu 1,4-Dihydropyridinen der allgemeinen Formel (I) umsetzen.

Verwendet man als Ausgangsstoffe Benzylidenacetessigsäuremethylester, Morpholinocyclohexen und Ammoniak, so kann der Reaktionsablauf durch folgendes Formelschema verdeutlicht werden:

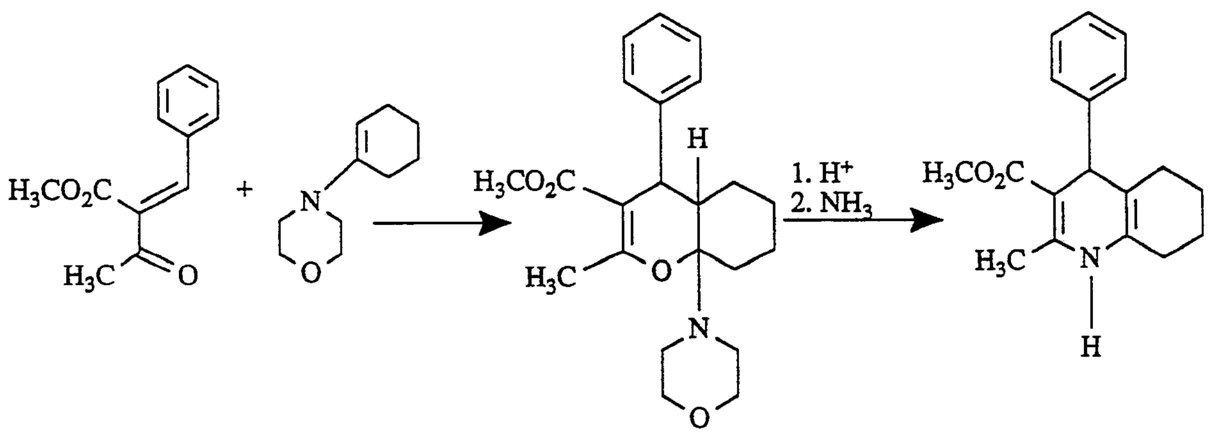


Die als Ausgangsstoffe verwendeten Benzylidenverbindungen der allgemeinen Formel (II) sind bekannt oder können nach literaturbekannten Methoden hergestellt werden [vgl. G. Jones "The Knoevenagel Kondensation" in Organic Reactions XV, 204 (1967), A. Dornow, W. Sassenberg, Liebigs Ann. Chem. *602*, 14 (1957)].

Die als Ausgangsstoffe verwendeten Enamine der allgemeinen Formel (III) sind bekannt oder können nach literaturbekannten Methoden hergestellt werden [vgl. J. Szmuszkovicz "Enamines", Adv. Org. Chem. *4*, 1 (1963), A. G. Cook "Enamines — Synthesis, Structure and Reactions", M. Dekker, New York 1969, H. O. House "Modern Synthetic Reactions", 2. Auflage, S. 570, W. A. Benjamin Inc. Menlo Park, 1972].

Die Amine der allgemeinen Formel (V) sind bekannt. Geeignete Säureadditionsprodukte können Salze der Amine (V) mit anorganischen oder organischen Säuren sein, wie beispielsweise Bromide, Chloride, Hydrogensulfate, Sulfate, Hydrogenphosphate, Acetate, Carbonate oder Hydrogencarbonate.

Die bei der Durchführung des erfindungsgemäßen Verfahrens entstehenden Zwischenprodukte IV sind neu. Es ist jedoch nicht erforderlich sie zu isolieren.

Als Lösungsmittel sowohl für die Herstellung der Zwischenprodukte als auch der Endprodukte kommen Wasser oder alle inerten organischen Lösungsmittel in Frage. Hierzu gehören vorzugsweise Alkohole wie Methanol, Ethanol, Propanol oder Isopropanol, Ether wie Diethylether, Dioxan, Tetrahydrofuran, Glykolmono- oder -dimethylether, Dimethylformamid, Dimethylsulfoxid, Acetonitril, Essigester, Hexamethylphosphorsäuretriamid, Pyridin, Halogenkohlenwasserstoffe wie Di-, Tri-, Tetrachlormethan,

Dichlorethan oder Trichlorethylen und aromatische Kohlenwasserstoffe wie Benzol, Toluol oder Xylol. Es ist jedoch auch möglich, Gemische der genannten Lösungsmittel einzusetzen.

Als Säuren können die üblichen anorganischen oder organischen Säuren verwendet werden. Hierzu gehören vorzugsweise Halogenwasserstoffe wie Chlorwasserstoff oder Bromwasserstoff, Schwefelsäure oder Phosphorsäure, oder organische Säuren wie Essigsäure, Propionsäure oder Weinsäure, oder Sulfonsäuren wie z.B. Methan-, Ethan-, Benzol- oder Toluolsulfonsäure.

Die Reaktionstemperaturen können in einem weiteren Bereich variiert werden. Im allgemeinen arbeitet man in einem Temperaturbereich von 0°C bis 200°C, bevorzugt von 10°C bis 150°C.

Das erfindungsgemäße Verfahren wird im allgemeinen bei Normaldruck durchgeführt. Es ist aber auch möglich, das Verfahren bei Unterdruck oder bei Überdruck durchzuführen.

Im allgemeinen setzt man auf 1 Mol Benzylidenverbindung II 0,5—5, bevorzugt 1—2 Mol Enamin III ein. Bezogen auf 1 Mol der Zwischenverbindung setzt man im allgemeinen 0,1—10 Mol, bevorzugt 0,5—5 Mol Säure ein. Das Amin V wird im allgemeinen in einer Menge von 0,1—10, bevorzugt 0,5—5 Mol bezogen auf ein Mol Zwischenverbindung verwendet.

Bei der erfindungsgemäßen Umsetzung der Zwischenprodukte zu den Dihydropyridinen sind verschiedene Verfahrensvarianten möglich. Bei Variante A wird zum Zwischenprodukt erst Säure dann Amin, bei Variante B erst Amin dann Säure und bei Variante C Amin und Säure in Form des Additionsproduktes der Säure an das Amin zugegeben.

Die erfindungsgemäßen hergestellten Stoffe der Formel (I) zeigen ein wertvolles, pharmakologisches Wirkspektrum. Sie beeinflussen den Kreislauf, die Kontraktionskraft des Herzens, den Gefäßtonus sowie den Tonus glatter Muskeln. Sie können daher in Arzneimitteln z.B. zur Behandlung von Kreislauferkranungen, coronaren Herzkrankheiten und Herzinsuffizienz eingesetzt werden. Darüber hinaus können sie den Blutzucker beeinflussen und so als Therapeutika für Stoffwechselerkrankungen dienen.

Die Herz- und Gefäßwirkungen wurden an isoliert perfundierten Herzen des Meerschweinchens gefunden. Dazu werden die Herzen von 250 bis 350 g schweren Albino Meerschweinchen verwendet. Die Tiere werden mit einem Schlag auf den Kopf getötet, der Thorax geöffnet, in die freipräparierte Aorta eine Metallkanüle eingebunden und der linke Vorhof eröffnet. Das Herz wird mit den Lungen aus dem Thorax herausgetrennt und über die Aortenkanüle an die Perfusionsapparatur bei laufender Perfusion angeschlossen. Die Lungen werden an den Lungenwurzeln abgetrennt. Als Perfusionsmedium dient Krebs-Henseleit-Lösung (1) (118,5 mmol/l NaCl, 4,75 mmol/l KCl, 1,19 mmol/l $KH_2PO_4$, 119 mmol/l $MgSO_4$, 25 mmol/l $NaHCO_3$, 0,013 mmol/l NaEDTA), deren $CaCl_2$ je nach Bedarf variiert wird, in der Regel jedoch 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$ zur Aufrechterhaltung des pH-Wertes von 7,4) begast. Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt und die isovolumetrischen Kontraktionen auf einem Schnellschreiber registriert (Opie, L., J. Physiol. *180* (1965) 529—541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdruckes eine Koronardilatation, eine Steigerung der linksventrikulären Druckamplitude einen Anstieg der Herzkontraktilität an. Die erfindungsgemäßen Verbindungen werden in geeigneten Verdünnungen in das Perfusionssystem kurz vor dem isolierten Herzen infundiert.

In der folgenden Tabelle sind die Wirkungen von einigen Beispielen auf Kontraktilität und Koronarwiderstand am isoliert perfundierten Meerschweinchenherzen aufgezeigt.

| | proz. Veränderung des Koronarwiderstandes | | | proz. Veränderung der Kontraktilität bei | | | |
|---|---|---|---|---|---|---|---|
| Bsp. Nr. | 10—7 | 10—6 | 10—5 | 10—7 | 10—6 | 10—5 | g/ml |
| 8 | −8 | −35 | −35 | 0 | −42 | −85 | |
| 9 | −23 | −43 | −48 | +13 | −74 | −98 | |
| 32 | −7 | −21 | −21 | −8 | −68 | −95 | |
| 33 | −4 | −29 | −41 | 0 | 0 | −52 | |

Die Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Fall wäßriger Suspensionen und/oder Elixieren, die für orale Anwendung gedacht sind, können die Wirkstoffe außer den obengenannten Hilfsstoffen mit verschiedenen Geschmackaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg, Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art, von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Herstellungsbeispiele

Beispiel 1

8a-Morpholino-4-(3-nitrophenyl)-2-methyl-4a,5,6,7,8,8a-hexahydro-4H-chromen-3-carbonsäureethylester

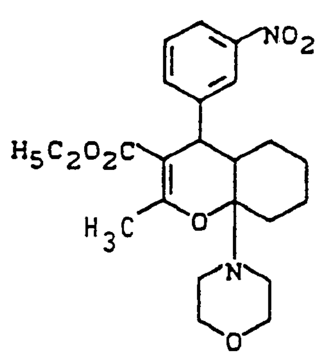

Eine Lösung von 0,033 mol 3-Nitrobenzylidenmethylessigsäureethylester und 0,033 mol Morpholinocyclohexen in 80 ml Ethanol wird 4 Stunden unter Rückfluß gekocht. Man engt ein, reibt mit Ether an, saugt ab und kristallisiert aus Ethanol um.

Schmelzpunkt: 133°C.

Beispiel 2

2-Methyl-4-(3-nitrophenyl)-1,4,5,6,7,8-hexahydrochinolin-3-carbonsäureethylester (Variante A)

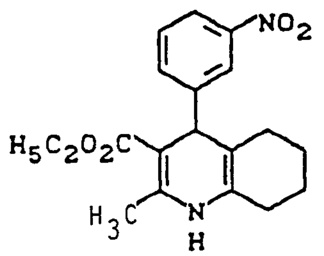

0,05 mol 8a-Morpholino-4-(3-nitrophenyl)-2-methyl-4a,5,6,7,8,8a-hexahydro-4H-chromen-3-carbonsäureethylester werden in einem Erlenmeyerkolben (Magnetrührer) mit etwa 100 ml Ethanol versetzt. Dann gibt man 200 ml einer Mischung von 50% Ethanol/50% konz. HCl hinzu und wartet die vollständige Lösung ab (evtl. von unlöslichem Material abfiltrieren).

Mit konz. Ammoniak stellt man dann alkalisch, läßt auf Raumtemperatur abkühlen (evtl. etwas Wasser zusetzen), saugt den Niederschlag ab und kristallisiert aus Ethanol um.

Schmelzpunkt: 131°C.

Analog Beispiel 1 wurden hergestellt:

Beispiel 3

3-Acetyl-2-methyl-8a-morpholino-4-(3-nitrophenyl)-4a,5,6,7,8,8a-hexahydro-4H-chromen

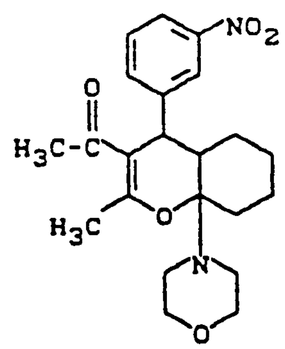

Schmelzpunkt: 164°C.

Beispiel 4

3-Acetyl-2-methyl-4-(3-nitrophenyl)-1,4,5,6,7,8-hexahydrochinolin (Variante A aus 5)

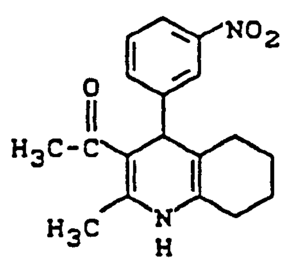

Schmelzpunkt: 161°C.

Beispiel 5

2-Methyl-8a-morpholino-3-nitro-4-(2-trifluormethylphenyl)-4a,5,6,7,8,8a-hexahydro-4H-chromen

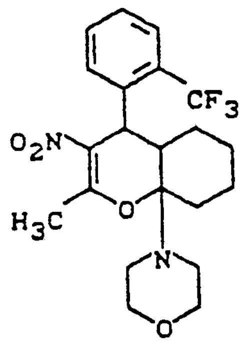

10,4 g (40 mmol) 2-Nitro-1-(2-trifluormethylphenyl)-buten-1-on-3 und 6,7 g (40 mmol) 1-Morpholinocyclohexen werden in 20 ml Ethanol bei Raumtemperatur zusammengegeben. Es findet eine exotherme Reaktion statt, nach kurzer Zeit kristallisiert das Produkt in blaßgelben Kristallen als Isomerengemisch.

Ausbeute: 13,4 g (77% der Theorie).

Schmelzpunkt: 133°C.

Beispiel 6

2-Methyl-3-nitro-4-(2-trifluormethylphenyl)-1,4,5,6,7,8-hexahydrochinolin (Variante B)

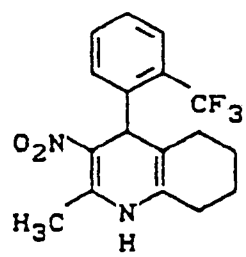

2 g (4,7 mmol) 2-Methyl-8a-morpholino-3-nitro-4-(2-trifluormethylphenyl)-4a,5,6,7,8,8a-hexahydro-4H-chromen werden in 15 ml Ethanol suspendiert und mit ca. 3 ml konzentrierter wäßriger Ammoniaklösung bei 30 bis 45°C in Lösung gebracht. Anschließend wird mit konzentrierter Salzsäure auf pH 3 eingestellt, die Lösung mit 5 ml Wasser verdünnt und 2 × mit je 10 ml Chloroform extrahiert. Nach Abdampfen des Chloroforms kristallisiert der Rückstand aus wenig Ethanol in gelben Kristallen.

Ausbeute: 580 mg (36% der Theorie).

Schmelzpunkt: 222°C (Zers.).

Beispiel 7

1,2-Dimethyl-3-nitro-4-(2-trifluormethylphenyl)-1,4,5,6,7,8-hexahydrochinolin (Variante C)

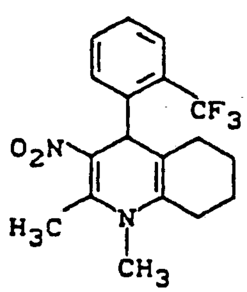

2 g (4,7 mmol) 2-Methyl-8a-morpholino-3-nitro-4-(2-trifluormethylphenyl)-4a,5,6,7,8,8a-hexahydro-4H-chromen werden in 15 ml Ethanol mit 2 g Methylammoniumchlorid 6 Stunden zum Rückfluß erhitzt. Nach dem Abkühlen saugt man die Salze ab, dampft die Lösung ein, nimmt den Rückstand in Chloroform auf und wäscht mit Wasser. Die organische Phase wird getrocknet und eingeengt, der Rückstand auf Kieselgel mit Chloroform mit 1% Methanol chromatographiert. Aus Ethanol erhält man intensiv gelbe Kristalle.

Ausbeute: 880 mg (52% der Theorie).

Schmelzpunkt: 149°C.

Analog den beschriebenen Verfahren wurden die in Tabelle 1 und 2 aufgeführten Beispiele hergestellt:

Tabelle 1

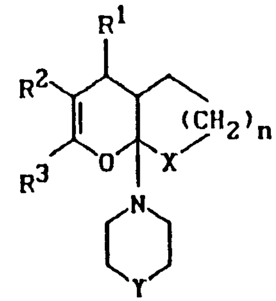

| Bsp.-Nr. | $R^1$ | $R^2$ | $R^3$ | n | X | Y | Schmelzpunkt [°C] |
|---|---|---|---|---|---|---|---|
| 8 | 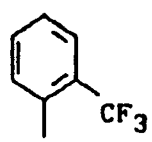 | $NO_2$ | $CH_3$ | 2 | Bindung | O | 128 |
| 9 | 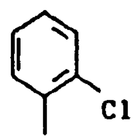 | $CO_2CH(CH_3)_2$ | $CH_3$ | 3 | Bindung | O | 120 |
| 10 | 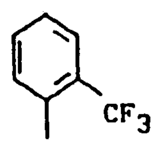 | $NO_2$ | $CH_3$ | 2 | >C=O | O | 132 |
| 11 | 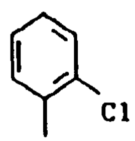 | $CO_2CH(CH_3)_2$ | $CH_3$ | 2 | >C=O | O | 172 |

| Bsp.-Nr. | $R^1$ | $R^2$ | $R^3$ | n | X | Y | Schmelzpunkt [°C] |
|---|---|---|---|---|---|---|---|
| 12 | 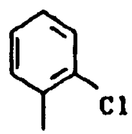 | $CO_2C_2H_5$ | $CF_3$ | 3 | Bindung | O | 116 |
| 13 | 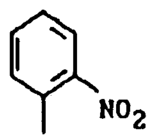 | $CO_2C_2H_5$ | $CH(CH_3)_2$ | 3 | Bindung | O | 158 |
| 14 | 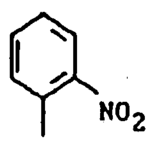 | $CO_2C_2H_5$ | $C_2H_5$ | 3 | Bindung | O | 124 |

| Bsp.-Nr. | $R^1$ | $R^2$ | $R^3$ | n | X | Y | Schmelzpunkt [°C] |
|---|---|---|---|---|---|---|---|
| 15 | 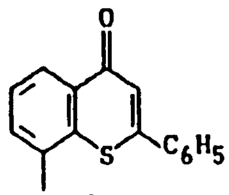 | $CO_2CH(CH_3)_2$ | $CH_2Cl$ | 3 | Bindung | O | 191 |
| 16 | 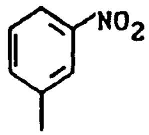 | $CO_2C_2H_5$ | $CH_3$ | 9 | Bindung | O | 122 |
| 17 | 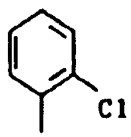 | $CO_2C_2H_5$ | $CH_2Cl$ | 3 | Bindung | O | 130 |
| 18 | 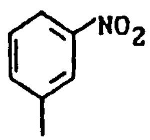 | $SO_2CH_3$ | $CH_3$ | 3 | Bindung | O | 153 |
| 19 | 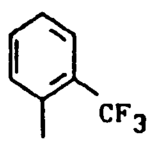 | $NO_2$ | $CH_3$ | 3 | Bindung | $CH_2$ | 116 |

| Bsp.-Nr. | $R^1$ | $R^2$ | $R^3$ | n | X | Y | Schmelzpunkt [°C] |
|---|---|---|---|---|---|---|---|
| 20 | 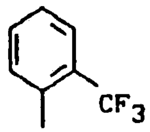 | $NO_2$ | $CH_3$ | 9 | Bindung | O | Harz |
| 21 | 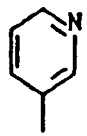 | $COCH_3$ | $CH_3$ | 3 | Bindung | O | 120 |
| 22 | 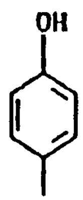 | CN | $CH_3$ | 3 | Bindung | O | 161 |
| 23 | 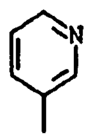 | $CO_2C_2H_5$ | $CH_3$ | 3 | Bindung | O | Harz |
| 24 | 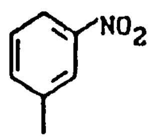 | $CO_2C_2H_5$ | $CH_3$ | 3 | Bindung | Bindung | 186 |
| 25 | 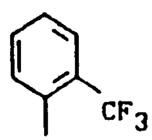 | $NO_2$ | $CH_3$ | 3 | Bindung | Bindung | 194 |

**Tabelle 2**

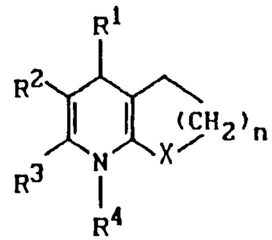

| Bsp.-Nr. | $R^1$ | $R^2$ | $R^3$ | $R^4$ | n | X | Schmelzpunkt [°C] |
|---|---|---|---|---|---|---|---|
| 26 | 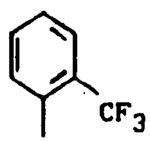 | $NO_2$ | $CH_3$ | H | 3 | Bindung | 222 |
| 27 | 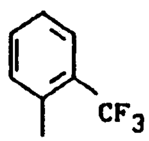 | $NO_2$ | $CH_3$ | $CH_3$ | 3 | Bindung | 149 |
| 28 | 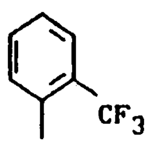 | $NO_2$ | $CH_3$ | H | 2 | >C=O | 188 |
| 29 | 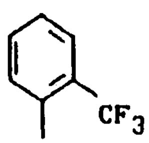 | $NO_2$ | $CH_3$ | $C_2H_5$ | 2 | >C=O | 168 |
| 30 | 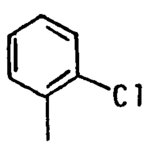 | $CO_2C_2N_5$ | $CF_3$ | H | 3 | Bindung | 116 |

| Bsp.-Nr. | $R^1$ | $R^2$ | $R^3$ | $R^4$ | n | X | Schmelzpunkt (°C) |
|---|---|---|---|---|---|---|---|
| 31 | 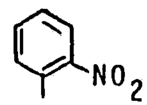 | $CO_2C_2H_5$ | $CH(CH_3)_2$ | H | 3 | Bindung | 158 |
| 32 | 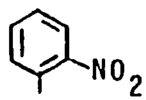 | $CO_2C_2H_5$ | $C_2H_5$ | H | 3 | Bindung | 124 |
| 33 | 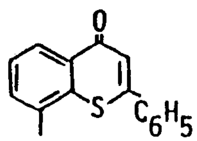 | $CO_2-CH(CH_3)_2$ | $CH_2Cl$ | H | 3 | Bindung | 191 |
| 34 | 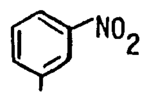 | $CO_2C_2H_5$ | $CH_3$ | H | 9 | Bindung | 122 |
| 35 | 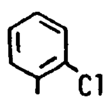 | $CO_2C_2H_5$ | $CH_2Cl$ | H | 3 | Bindung | 130 |
| 36 | 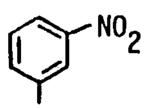 | $SO_2CH_3$ | $CH_3$ | H | 3 | Bindung | 153 |

| Bsp.-Nr. | $R^1$ | $R^2$ | $R^3$ | $R^4$ | n | X | Schmelzpunkt (°C) |
|---|---|---|---|---|---|---|---|
| 37 | 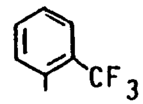 | $SO_2CH_3$ | $CH_3$ | H | 3 | Bindung | 116 |
| 38 | 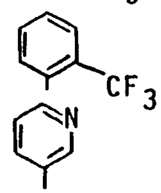 | $NO_2$ | $CH_3$ | H | 9 | Bindung | Harz |
| 39 | | $COCH_3$ | $CH_3$ | H | 3 | Bindung | 120 |
| 40 | 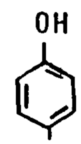 | CN | $CH_3$ | H | 3 | Bindung | 161 |
| 41 | 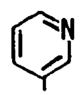 | $CO_2C_2H_5$ | $CH_3$ | H | 3 | Bindung | Harz |
| 42 | 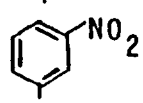 | $CO_2C_2H_5$ | $CH_3$ | H | 3 | Bindung | Harz |
| 43 | 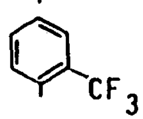 | $NO_2$ | $CH_3$ | H | 3 | Bindung | Harz |

**Patentansprüche**

1. Verfahren zur Herstellung von 1,4-Dihydropyridinen der allgemeinen Formel (I)

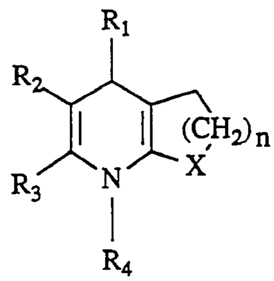

(I)

in welcher

$R_1$ für Phenyl, Thienyl, Furyl, Pyridyl, Pyrimidyl, Benzoxadiazolyl oder Thiochromenyl steht, wobei die genannten Reste gegebenenfalls 1 bis 2 gleiche oder verschiedene Substituenten aus der Gruppe $C_1$—$C_4$-Alkyl, $C_1$—$C_4$-Alkoxy, $C_1$—$C_4$-Alkylthio, Fluor, Chlor, Nitro, Cyano, Hydroxy, Trifluormethyl, Trifluormethoxy, Difluormethoxy, Fluormethoxy, Phenyl, Benzyl, Benzyloxy oder Benzylthio enthalten,

$R_2$ für Wasserstoff, Nitro, Cyano, $C_1$—$C_4$-Alkylsulfonyl oder für die Gruppe

$$-\underset{\underset{O}{\|}}{C}-R_5 \text{ steht}$$

wobei

$R_5$ für geradkettiges oder verzweigtes $C_1$—$C_4$-Alkyl steht, oder für eine Gruppe der Formel —O—$R_6$ steht, wobei

$R_6$ geradkettiges, verzweigtes oder cyclisches $C_1$—$C_6$-Alkyl darstellt, das gegebenenfalls durch ein Sauerstoffatom in der Kette unterbrochen ist und das gegebenenfalls substituiert ist durch ein oder mehrere Fluor, Hydroxy, Cyano, Phenyl oder Benzylmethylamino,

$R_3$ für geradkettiges oder verzweigtes $C_1$—$C_4$-Alkyl steht, das gegebenenfalls substituiert ist durch Hydroxy, Chlor, Brom oder Acetyloxy,

$R_4$ für Wasserstoff oder geradkettiges oder verzweigtes $C_1$—$C_4$-Alkyl steht,

X für die Gruppe =C=O oder für eine direkte Bindung steht und

n für eine Zahl von 1 bis 12 steht,

in Form ihrer Isomeren, Isomerengemische, Racematen und optischen Antipoden sowie ihrer physiologisch unbedenklichen Salze, dadurch gekennzeichnet, daß man Verbindungen der allgemeinen Formel (IV)

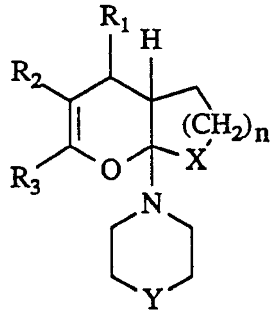

(IV)

in welcher

$R_1$, $R_2$, $R_3$, X und n die oben angegebenen Bedeutung haben, und

Y für eine direkte Bindung, für Sauerstoff, NH, Methylen oder Ethylen steht mit Aminen der allgemeinen Formel (V)

$$R_4—NH_2 \qquad (V)$$

in welcher

$R_4$ die oben angegebene Bedeutung hat

oder mit deren Additionsprodukten in Anwesenheit von Säuren und gegebenenfalls in Anwesenheit von Wasser oder inerten organischen Lösungsmitteln umsetzt.

2. Verbindungen der allgemeinen Formel (IV)

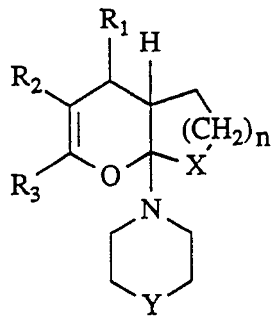

(IV)

in welcher

$R_1$ für Phenyl, Thienyl, Furyl, Pyridyl, Pyrimidyl, Benzoxadiazolyl oder Thiochromenyl steht, wobei die genannten Reste gegebenenfalls 1 bis 2 gleiche oder verschiedene Substituenten aus der Gruppe $C_1$—$C_4$-Alkyl, $C_1$—$C_4$-Alkoxy, $C_1$—$C_4$-Alkylthio, Fluor, Chlor, Nitro, Cyano, Hydroxy, Trifluormethyl, Trifluormethoxy, Difluormethoxy, Fluormethoxy, Phenyl, Benzyl, Benzyloxy oder Benzylthio enthalten,

$R_2$ für Wasserstoff, Nitro, Cyano, $C_1$—$C_4$-Alkylsulfonyl oder für die Gruppe

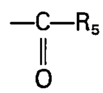 steht

wobei

$R_5$ für geradkettiges oder verzweigtes $C_1$—$C_4$-Alkyl steht, oder für eine Gruppe der Formel —O—$R_6$ steht, wobei

$R_6$ geradkettiges, verzweigtes oder cyclisches $C_1$—$C_6$-Alkyl darstellt, das gegebenenfalls durch ein Sauerstoffatom in der Kette unterbrochen ist und das gegebenenfalls substituiert ist durch ein oder mehrere Fluor, Hydroxy, Cyano, Phenyl oder Benzylmethylamino,

$R_3$ für geradkettiges oder verzweigtes $C_1$—$C_4$-Alkyl steht, das gegebenenfalls substituiert ist durch Hydroxy, Chlor, Brom oder Acetyloxy,

X für die Gruppe

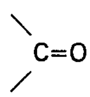

oder für eine direkte Bindung steht, und

n für eine Zahl von 1 bis 12 steht, und

Y für eine direkte Bindung, für Sauerstoff, NH, Methylen oder Ethylen steht.

3. Verfahren zur Herstellung von Verbindungen der allgemeinen Formel (IV)

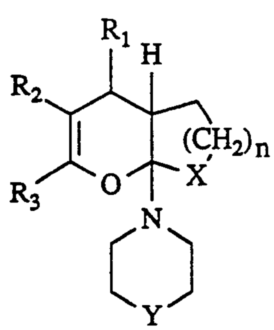

(IV)

in welcher

$R_1$ für Phenyl, Thienyl, Furyl, Pyridyl, Pyrimidyl, Benzoxadiazolyl oder Thiochromenyl steht, wobei die genannten Reste gegebenenfalls 1 bis 2 gleiche oder verschiedene Substituenten aus der Gruppe $C_1$—$C_4$-

Alkyl, $C_1$—$C_4$-Alkoxy, $C_1$—$C_4$-Alkylthio, Fluor, Chlor, Nitro, Cyano, Hydroxy, Trifluormethyl, Trifluormethoxy, Difluormethoxy, Fluormethoxy, Phenyl, Benzyl, Benzyloxy oder Benzylthio enthalten,

$R_2$ für Wasserstoff, Nitro, Cyano, $C_1$—$C_4$-Alkylsulfonyl oder für die Gruppe

$$-\underset{\underset{O}{\parallel}}{C}-R_5 \text{ steht}$$

wobei

$R_5$ für geradkettiges oder verzweigtes $C_1$—$C_4$-Alkyl steht, oder für eine Gruppe der Formel —O—$R_6$ steht, wobei

$R_6$ geradkettiges, verzweigtes oder cyclisches $C_1$—$C_6$-Alkyl darstellt, das gegebenenfalls durch ein Sauerstoffatom in der Kette unterbrochen ist und das gegebenenfalls substituiert ist durch ein oder mehrere Fluor, Hydroxy, Cyano, Phenyl oder Benzylmethylamino,

$R_3$ für geradkettiges oder verzweigtes $C_1$—$C_4$-Alkyl steht, das gegebenenfalls substituiert ist durch Hydroxy, Chlor, Brom oder Acetyloxy,

X für die Gruppe

$$>C=O$$

oder für eine direkte Bindung steht, und

n für eine Zahl von 1 bis 12 steht, und

Y für eine direkte Bindung, für Sauerstoff, NH, Methylen oder Ethylen steht, dadurch gekennzeichnet, daß man Benzylidenverbindungen der allgemeinen Formel (II)

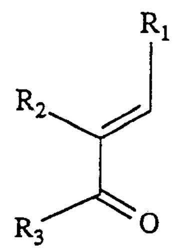

(II)

in welcher

$R_1$—$R_3$ die oben angegebene Bedeutung haben,

und Enamine der allgemeinen Formel (III)

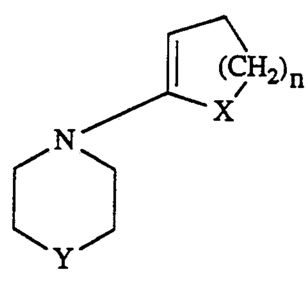

(III)

in welcher

X, Y und n die oben angegebene Bedeutung haben, gegebenenfalls in Gegenwart von Wasser und/oder inerten organischen Lösungsmitteln bei Temperaturen zwischen 10°C und 150°C umsetzt.

4. Verwendung von Verbindungen der allgemeinen Formel (IV) gemäß Anspruch 2 bei der Herstellung von Dihydropyridinderivaten.

**Revendications**

1. Procédé de production de 1,4-dihydropyridines de formule générale (I)

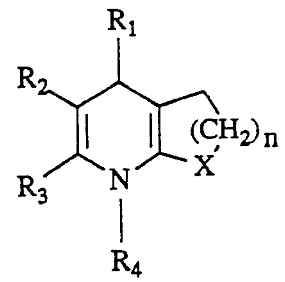

(I)

dans laquelle

$R_1$ est un reste phényle, thiényle, furyle, pyridyle, pyrimidyle, benzoxadiazolyle ou thiochroményle, les restes mentionnés contenant le cas échéant 1 ou 2 substituants identiques ou différents choisis dans le groupe des substituants alkyle en $C_1$ à $C_4$, alkoxy en $C_1$ à $C_4$, alkylthio en $C_1$ à $C_4$, fluoro, chloro, nitro, cyano, hydroxy, trifluorométhyle, trifluorométhoxy, difluorométhoxy, fluorométhoxy, phényle, benzyle, benzyloxy ou benzylthio,

$R_2$ représente l'hydrogène, un groupe nitro, cyano, alkylsulfonyle en $C_1$ à $C_4$ ou le groupe

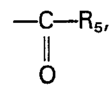

dans lequel

$R_5$ est un reste alkyle en $C_1$ à $C_4$ à chaîne droite ou ramifiée ou un groupe de formule $—O—R_6$, dans lequel

$R_6$ est un reste alkyle en $C_1$ à $C_6$ à chaîne droite ou ramifiée ou cyclique, dont la chaîne est éventuellement interrompue par un atome d'oxygène et qui est éventuellement substitué par un ou plusieurs radicaux fluoro, hydroxy, cyano, phényle ou benzylméthylamino,

$R_3$ est un groupe alkyle en $C_1$ à $C_4$ à chaîne droite ou ramifiée qui est substitué éventuellement par un radical hydroxy, chloro, bromo ou acétyloxy,

$R_4$ est l'hydrogène ou un groupe alkyle en $C_1$ à $C_4$ à chaîne droite ou ramifiée,

X est le groupe =C=O ou une liaison simple et

n est un nombre de 1 à 12,

sous forme de leurs isomères, de leurs mélanges d'isomères, de leurs racémates et de leurs antipodes optiques ainsi que de leurs sels acceptables du point de vue physiologique, caractérisé en ce qu'on fait réagir des composés de formule générale (IV)

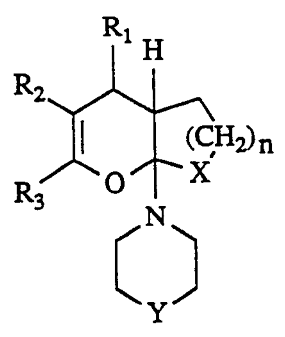

(IV)

dans laquelle

$R_1$, $R_2$, $R_3$, X et n ont la définition indiquée ci-dessus et

Y est une liaison simple, l'oxygène, un groupe NH, méthylène ou éthylène, avec des amines de formule générale (V)

$$R_4—NH_2 \qquad (V)$$

dans laquelle

$R_4$ a la définition indiquée ci-dessus

ou avec leurs produits d'addition en présence d'acides et le cas échéant en présence d'eau ou de solvants organiques inertes.

2. Composés de formule générale (IV)

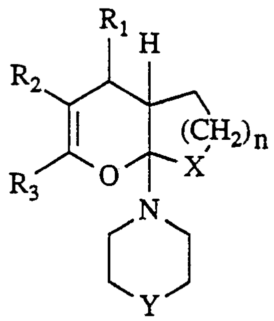

(IV)

dans laquelle

$R_1$ est un reste phényle, thiényle, furyle, pyridyle, pyrimidyle, benzoxadiazolyle ou thiochroményle, les restes mentionnés contenant le cas échéant 1 ou 2 substituants identiques ou différents choisis dans le groupe des substituants alkyle en $C_1$ à $C_4$, alkoxy en $C_1$ à $C_4$, alkylthio en $C_1$ à $C_4$, fluoro, chloro, nitro, cyano, hydroxy, trifluorométhyle, trifluorométhoxy, difluorométhoxy, fluorométhoxy, phényle, benzyle, benzyloxy ou benzylthio,

$R_2$ représente l'hydrogène, un groupe nitro, cyano, alkylsulfonyle en $C_1$ à $C_4$ ou le groupe

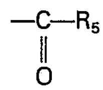

dans lequel

$R_5$ est un reste alkyle à chaîne droite ou ramifiée en $C_1$ à $C_4$ ou un groupe de formule $-O-R_6$ dans lequel

$R_6$ est un reste alkyle en $C_1$ à $C_6$ à chaîne droite, ramifiée ou cyclique, dont la chaîne est interrompue le cas échéant par un atome d'oxygène et qui est éventuellement substitué par un ou plusieurs substituants fluoro, hydroxy, cyano, phényle ou benzylméthylamino,

$R_3$ est un groupe alkyle en $C_1$ à $C_4$ à chaîne droite ou ramifiée, qui est substitué le cas échéant par un radical hydroxy, chloro, bromo ou acétyloxy,

X représente le groupe

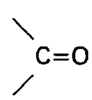

ou un liaison simple et

n est un nombre de 1 à 12 et

Y est un liaison simple, l'oxygène, un groupe NH, méthylène ou éthylène.

3. Procédé de production de composés de formule générale (IV)

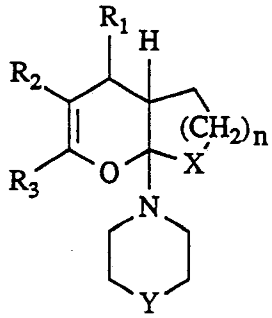

(IV)

dans laquelle

$R_1$ est un reste phényle, thiényle, furyle, pyridyle, pyrimidyle, benzoxadiazolyle ou thiochroményle, les

restes mentionnés contenant le cas échéant 1 ou 2 substituants identiques ou différents choisis dans le groupe des substituants alkyle en $C_1$ à $C_4$, alkoxy en $C_1$ à $C_4$, alkylthio en $C_1$ à $C_4$, fluoro, chloro, nitro, cyano, hydroxy, trifluorométhyle, trifluorométhoxy, difluorométhoxy, fluorométhoxy, phényle, benzyle, benzyloxy ou benzylthio,

$R_2$ est l'hydrogène, un groupe nitro, cyano, alkylsulfonyle en $C_1$ à $C_4$ ou le groupe

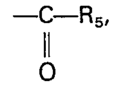

dans lequel

$R_5$ est un reste alkyle à chaîne droite ou ramifiée en $C_1$ à $C_4$ ou un groupe de formule —O—$R_6$ dans laquelle

$R_6$ est un reste alkyle en $C_1$ à $C_6$ à chaîne droite, ramifiée ou cyclique, dont la chaîne est interrompue le cas échéant par un atome d'oxygène et qui est éventuellement substitué par un ou plusieurs substituants fluoro, hydroxy, cyano, phényle ou benzylméthylamino,

$R_3$ est un groupe alkyle en $C_1$ à $C_4$ à chaîne droite ou ramifiée, qui est substitué le cas échéant par un radical hydroxy, chloro, bromo ou acétyloxy,

X représente le groupe

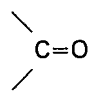

ou un liaison simple et

n est un nombre de 1 à 12 et

Y est un liaison simple, l'oxygène, un groupe NH, méthylène ou éthylène,

caractérisé en ce qu'on fait réagir des composés benzylidéniques de formule générale (II)

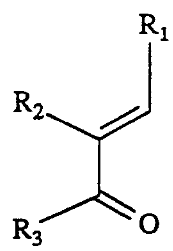

(II)

dans laquelle

$R_1$ à $R_3$ ont la définition indiquée ci-dessus,

et des énamines de formule générale (III)

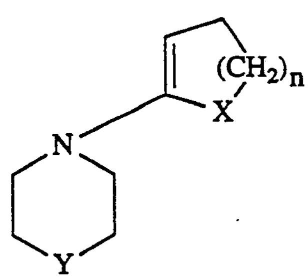

(III)

dans laquelle

X, Y et n ont la définition indiquée ci-dessus, le cas échéant en présence d'eau et/ou de solvants organiques inertes, à des températures comprises entre 10°C et 150°C.

4. Utilisation de composés de formule générale (IV) suivant la revendication 2 dans la préparation de dérivés de dihydropyridines.

**Claims**

1. Process for preparing 1,4-dihydropyridines of the general formula (I)

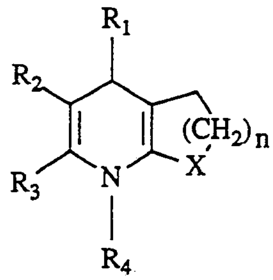

(I)

in which

$R_1$ represents phenyl, thienyl, furyl, pyridyl, pyrimidyl, benzoxadiazolyl or thiochromenyl, the radicals mentioned optionally containing 1 or 2 identical or different substituents from the group comprising $C_1$—$C_4$-alkyl, $C_1$—$C_4$-alkoxy, $C_1$—$C_4$-alkylthio, fluorine, chlorine, nitro, cyano, hydroxyl, trifluoromethyl, trifluoromethoxy, difluoromethoxy, fluoromethoxy, phenyl, benzyl, benzyloxy and benzylthio,

$R_2$ represents hydrogen, nitro, cyano or $C_1$—$C_4$-alkylsulphonyl, or represents the group

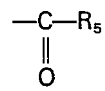

wherein

$R_5$ represents straight-chain or branched $C_1$—$C_4$-alkyl, or represents a group of the formula —O—$R_6$, wherein

$R_6$ represents straight-chain, branched or cyclic $C_1$—$C_6$-alkyl, which is optionally interrupted in the chain by an oxygen atom and which is optionally substituted by one or more fluorine, hydroxyl, cyano, phenyl or benzylmethylamino groups,

$R_3$ represents straight-chain or branched $C_1$—$C_4$-alkyl, which is optionally substituted by hydroxyl, chlorine, bromine or acetyloxy,

$R_4$ represents hydrogen, or represents straight-chain or branched $C_1$—$C_4$-alkyl,

X represents the group =C=O, or represents a direct bond and

n represents a number from 1 to 12,

in the form of their isomers, isomer mixtures, racemates and optical antipodes and their physiologically acceptable salts, characterized in that compounds of the general formula (IV)

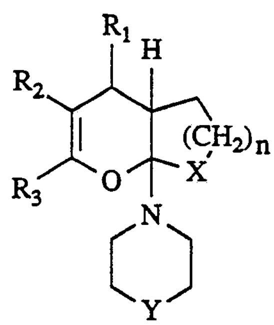

(IV)

in which

$R_1$, $R_2$, $R_3$, X and n have the abovementioned meaning, and

Y represents a direct bond, or represents oxygen, NH, methylene or ethylene are reacted with amines of the general formula (V)

$$R_4—NH_2 \qquad (V)$$

in which

$R_4$ has the abovementioned meaning,

or with addition products thereof, in the presence of acids and if appropriate in the presence of water or inert organic solvents.

2. Compounds of the general formula (IV)

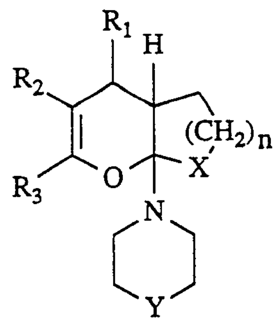

(IV)

in which

$R_1$ represents phenyl, thienyl, furyl, pyridyl, pyrimidyl, benzoxadiazolyl or thiochromenyl, the radicals mentioned optionally containing 1 or 2 identical or different substituents from the group comprising $C_1$—$C_4$-alkyl, $C_1$—$C_4$-alkoxy, $C_1$—$C_4$-alkylthio, fluorine, chlorine, nitro, cyano, hydroxyl, trifluoromethyl, trifluoromethoxy, difluoromethoxy, fluoromethoxy, phenyl, benzyl, benzyloxy and benzylthio,

$R_2$ represents hydrogen, nitro, cyano or $C_1$—$C_4$-alkylsulphonyl, or represents the group

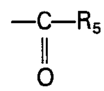

wherein

$R_5$ represents straight-chain or branched $C_1$—$C_4$-alkyl, or represents a group of the formula —O—$R_6$, wherein

$R_6$ represents straight-chain, branched or cyclic $C_1$—$C_6$-alkyl, which is optionally interrupted in the chain by an oxygen atom and which is optionally substituted by one or more fluorine, hydroxyl, cyano, phenyl or benzylmethylamino groups,

$R_3$ represents straight-chain or branched $C_1$—$C_4$-alkyl, which is optionally substituted by hydroxyl, chlorine, bromine or acetyloxy,

X represents the group

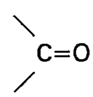

or represents a direct bond and

n represents a number from 1 to 12, and

Y represents a direct bond, or represents oxygen, NH, methylene or ethylene.

3. Process for preparing compounds of the general formula (IV)

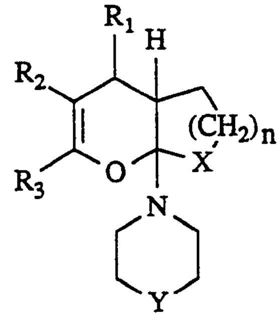

(IV)

in which

$R_1$ represents phenyl, thienyl, furyl, pyridyl, pyrimidyl, benzoxadiazolyl or thiochromenyl, the radicals

mentioned optionally containing 1 or 2 identical or different substituents from the group comprising $C_1$—$C_4$-alkyl, $C_1$—$C_4$-alkoxy, $C_1$—$C_4$-alkylthio, fluorine, chlorine, nitro, cyano, hydroxyl, trifluoromethyl, trifluoromethoxy, difluoromethoxy, fluoromethoxy, phenyl, benzyl, benzyloxy and benzylthio,

$R_2$ represents hydrogen, nitro, cyano or $C_1$—$C_4$-alkylsulphonyl, or represents the group

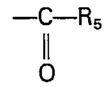

wherein

$R_5$ represents straight-chain or branched $C_1$—$C_4$-alkyl, or represents a group of the formula —O—$R_6$, wherein

$R_6$ represents straight-chain, branched or cyclic $C_1$—$C_6$-alkyl, which is optionally interrupted in the chain by an oxygen atom and which is optionally substituted by one or more fluorine, hydroxyl, cyano, phenyl or benzylmethylamino groups,

$R_3$ represents straight-chain or branched $C_1$—$C_4$-alkyl, which is optionally substituted by hydroxyl, chlorine, bromine or acetyloxy,

X represents the group

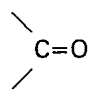

or represents a direct bond and

n represents a number from 1 to 12, and

Y represents a direct bond, or represents oxygen, NH, methylene or ethylene,

characterized in that benzylidene compounds of the general formula (II)

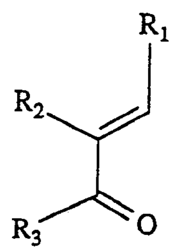

(II)

in which

$R_1$—$R_3$ have the abovementioned meaning, and enamines of the general formula (III)

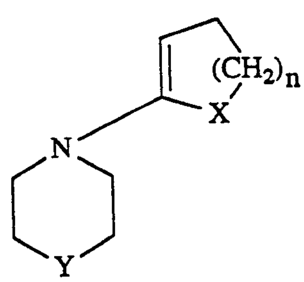

(III)

in which

X, Y and n have the abovementioned meaning are reacted, if appropriate in the presence of water and/or inert organic solvents at temperatures between 10°C and 150°C.

4. Use of compounds of the general formula (IV) according to Claim 2 in the preparation of dihydropyridine derivatives.